# EUROPÄISCHE PATENTSCHRIFT

(11) **EP 1 348 685 B1**
(45) Veröffentlichungstag und Bekanntmachung des Hinweises auf die Patenterteilung: **13.12.2006**
(21) Anmeldenummer: 03002874.0
(22) Anmeldetag: 08.02.2003
(51) Int. Cl.: C07C 29/151, C01B 3/22

(54) **Verfahren zur Erzeugung von Kohlenmonoxid und Methanol**
Process for the preparation of carbon monoxide and methanol
Procédé de préparation de monoxyde de carbone et de méthanol

(30) Priorität: 27.03.2002 DE 10214003
(43) Veröffentlichungstag der Anmeldung: 01.10.2003
(73) Patentinhaber: Lurgi AG, 60295 Frankfurt am Main (DE)
(72) Erfinder: Grobys, Mauricio, The Woodlands, TX 77381 (US); Göhna, Hermann, 65812 Bad Soden (DE); Wurzel, Thomas, 60439 Frankfurt am Main (DE)

(56) Entgegenhaltungen:
- EP-A- 0 533 231
- WO-A-01/32594
- GB-A- 1 523 797
- US-A- 5 079 267

## Beschreibung

Die Erfindung betrifft ein Verfahren zum Erzeugen von Methanol aus flüssigen oder gasförmigen Einsatzstoffen, wobei man aus diesen Einsatzstoffen unter Zugabe von Sauerstoff und Wasserdampf mittels katalytischer oder nichtkatalytischer partieller Oxidation ein Synthesegas mit einer Gaszusammensetzung herstellt, dass eine Stöchiometriezahl SN < 2 aufweist, und dieses Synthesegas mit Wasserstoff anreichert und/oder die kohlenstoffhaltigen Komponenten Kohlenmonoxid und/oder Kohlendioxid abzieht, um die für die Methanolsynthese benötigte Stöchiometriezahl von 2,0 bis 2,2 zu erreichen.

Weltweite Initiativen zum Klimaschutz und zur Reduzierung des globalen Ausstoßes an Treibhausgasen machen es erforderlich, verfahrenstechnische Anlagen zu konzipieren, die eine möglichst geringe Emission an Kohlendioxid aufweisen.

Es ist allgemeiner Stand der Technik (z.B. DE-C-2056824, EP-A-0533231), durch verschiedene Verfahren die für die Methanolsynthese benötigte Stöchiometriezahl einzustellen.
In der US-B-6,232,352 wird ein Verfahren beschrieben, bei dem Kohlenmonoxid und Methanol für die Essigsäureherstellung bereit gestellt werden. Hier erfolgt die Synthesegaserzeugung mittels Dampfreformierung.

Ausgehend von diesem Stand der Technik liegt der Erfindung die Aufgabe zugrunde, ein kostengünstiges Verfahren zur Herstellung von Methanol zu entwickeln, bei dem die Kohlendioxidemission reduziert oder sogar ganz vermieden wird.

Erfindungsgemäß wird die Aufgabe bei dem eingangs genannten Verfahren dadurch gelöst, dass einem autotherm betriebenen Reaktor zur Erzeugung von Synthesegas flüssige oder gasförmige Einsatzstoffe, Sauerstoff und Wasserdampf zugeführt wird, ein Teilstrom des Synthesegases abgezweigt und das darin enthaltene Kohlendioxid durch eine Kohlendioxid-Abtrennung entfernt wird, dass dieses Kohlendioxid ganz oder teilweise in die Synthesegaserzeugung zurückgeführt wird, dass das aus der Kohlendioxid-Abtrennung abgezogene kohlendioxidfreie Gas einer Trennung unterworfen wird, wobei Kohlenmonoxid produziert wird und der abgetrennte Wasserstoff dem Synthesegas ganz oder teilweise wieder zugeführt wird und dass das mit Wasserstoff angereicherte Synthesegas in der Methanolsynthese zu Methanol und Purgegas umgesetzt wird.

Mit diesem Verfahren ist es im Vergleich zu herkömmlichen Verfahren möglich, eine Anlage zur Herstellung von Methanol zu betreiben, die keine oder nur eine geringe Emission an Kohlendioxid erzeugt. Das in die Synthesegaserzeugung zurückgeführte Kohlendioxid wird im Reaktor teilweise in Kohlenmonoxid umgewandelt. Mit dem abgetrennten Wasserstoff wird die für die Methanolproduktion notwendige Stöchiometrie eingestellt und reines Kohlenmonoxid kann als Produkt gewonnen werden. Bei vollständigem Recycling des Kohlendioxids und des Purgegases ist es möglich, Methanol ohne Kohlendioxid-Emission zu produzieren. Wird nur teilweise das Kohlendioxid und das Purgegas zurückgeführt, wird die Kohlendioxid-Emission trotzdem deutlich reduziert.
Die Anlagenkonfiguration ermöglicht es, bei der Zuführung von externem Kohlendioxid Methanol zu produzieren, wobei der Anlagen-Verbrauch an Kohlendioxid größer ist als der Kohlendioxidausstoß.
Die Kohlenmonoxid-Abtrennung kann mittels kryogener Trennung oder durch Membranen erfolgen.
Der Kohlendioxid-Ausstoß des Vorwärmers kann dadurch vermindert werden, dass ein Teil des aus der Gastrennung entstehenden Wasserstoffs zur Unterfeuerung des Vorwärmers verwendet wird.
Die Verringerung bzw. Vermeidung von kohlendioxid-haltigen Abgasen des Vorwärmers wird ebenfalls dadurch erreicht, dass das Purgegas der Methanolsynthese mittels einer Gastrennung in eine wasserstoffreiche und eine kohlenstoffreiche Fraktion aufgetrennt wird und die wasserstoffreiche Fraktion zur Unterfeuerung des Vorwärmers verwendet wird. Die kohlenstoffreiche Fraktion wird zur partiellen Oxidation vor den Vorwärmer zurückgeführt.

Das Kohlenmonoxid, das aus der der Kohlendioxid-Abtrennung nachfolgenden Gastrennung entsteht, kann mit einem Teil des Methanols direkt weiterverarbeitet werden. Hier bietet sich bevorzugt eine Anlage zur Herstellung von Essigsäure an.
Die katalytische partielle Oxidation, die ohne indirekte Beheizung arbeitet, ist bekannt und z. B. in Ullmanns Encyclopedia of Industrial Chemistry, 5. Auflage, Band A 12, Seiten 202 bis 204 beschrieben.
Die flüssigen oder gasförmigen Einsatzstoffe, der Wasserdampf, sowie das Oxidationsmittel werden dem Reaktor vorzugsweise vorgewärmt zugeführt. Als Einsatzstoffe können bevorzugt Erdgas, aber auch Flüssiggas oder Raffineriegas verwendet werden. Üblicherweise führt man dem Brenner des Reaktors technisch reinen Sauerstoff zu, um den Gehalt an Inertgas im rohen Synthesegas möglichst niedrig zu halten. Es ist aber auch wahlweise die Verarbeitung eines Sauerstoff-Luft-Gemisches möglich. Die Zufuhr an Wasserdampf liegt üblicherweise im Bereich von 0,2 bis 3,0 Mol bezogen auf den molaren Kohlenstoffgehalt des Erdgases.

Für die Methanolsynthese kommen an sich bekannte Verfahren infrage, insbesondere die mit wassergekühltem Röhrenreaktor oder mit einem adiabat betriebene Festbettreaktor oder einer Kombination von einem wassergekühltem und einem gasgekühlten Reaktor arbeiten.

Eine weitere Verbesserung wird dadurch erreicht, dass zwischen den Stufen des Vorwärmers ein Vorreaktor eingesetzt wird, der mit einem aktiven Nickelkatalysator arbeitet, und höhere Kohlenwasserstoffe des Erdgases, wie z.B. Ethan oder Propan, in Methan, Kohlenmonoxid und Wasserstoff umwandelt. Dieses vorreagierte Gas läßt sich ohne Crackreaktion beliebig hoch vorwärmen.

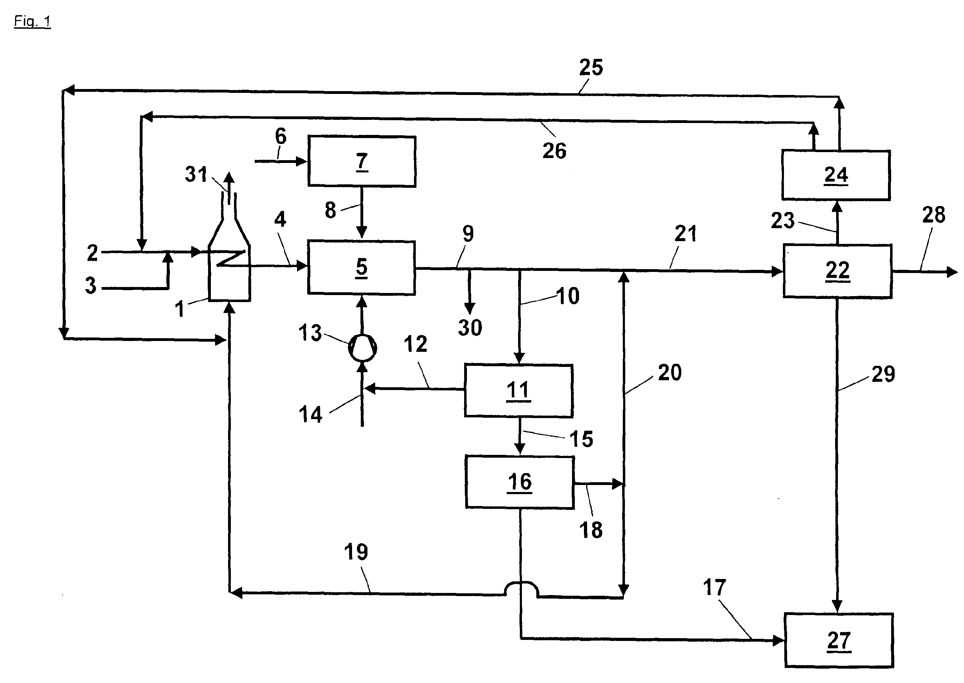

Ausgestaltungsmöglichkeiten des Verfahrens werden mit Hilfe der Zeichnung beispielhaft erläutert. Die Zeichnung zeigt ein Fliessschema des Verfahrens.

In einem gefeuerten Vorwärmer (1) werden über Leitung (2) Erdgas und über Leitung (3) Wasserdampf zugeführt und vorgewärmt und einem Reaktor (5) für partielle Oxidation über Leitung (4) zugeführt. Luft wird über Leitung (6) durch eine Trennung (7) zu Sauerstoff aufbereitet und über Leitung (8) ebenfalls dem Reaktor (5) zugeführt. In diesem Reaktor (5) wird mittels partieller Oxidation ein Synthesegas hergestellt, das im wesentlichen aus H₂, CO und CO₂ besteht und eine Stöchiometriezahl SN < 2 aufweist (SN = (H₂-CO₂)/(CO+CO₂) mol/mol). Dieses Gas wird über Leitung (9) aus dem Reaktor (5) abgezogen. Über Leitung (30) wird nach einer Abkühlung der nicht umgesetzte, kondensierte Wasserdampf abgezogen. Ein Teil des Synthesegases wird über Leitung (10) abgezweigt und das darin enthaltene CO₂ durch eine Abtrennung (11), üblicherweise eine Gaswäsche, entfernt. Das CO₂ aus der Abtrennung (11) wird über Leitung (12) einem Kompressor (13) und danach wieder dem Synthesegasreaktor (5) zugeführt. An dieser Stelle kann auch CO₂ aus externen Quellen (14) zugeführt werden.
Das CO₂-freie Gas aus der Gaswäsche (11) wird über Leitung (15) einer kryogenen Trennung (16) zugeführt. Das darin hergestellte Kohlenmonoxid besitzt typischerweise eine Reinheit von >98 Vol.-% CO. Geringere Reinheiten sind in Abhängigkeit der nachfolgenden CO-Verwendung möglich. Das bei der Tieftemperaturdestillation anfallende wasserstoffreiche Gas (18) wird in die Leitungen (19) und (20) aufgeteilt. Dabei kann das wasserstoffreiche Gas (18) ganz oder teilweise wieder dem Hauptsynthesegasstrom (9) über Leitung (20) zugeführt werden. Ab dem Einleitungspunkt des wasserstoffreichen Gases über Leitung (20) in den Hauptsynthesegasstrom (9) wird die für die Methanolsynthese notwendige Gaszusammensetzung mit der Stöchiometriezahl von 2,0 bis 2,2 erreicht. Dieses für die Methanolerzeugung erforderliche Synthesegas wird über Leitung (21) anschließend in der Methanolsynthese (22) zu Methanol umgesetzt. Im Falle, dass nur ein Teilstrom (20) des wasserstoffreichen Gases zum Hauptsynthesegasstrom (9) zurückgeführt wird, gelangt die restliche wasserstoffreiche Gasfraktion über Leitung (19) zur Unterfeuerung des Vorwärmers (1).
Das Purgegas (23) aus der Methanolsynthese (22) wird in der Gastrennung (24) in eine wasserstoffreiche (25) und eine kohlenstoffreiche Fraktion (26) getrennt. Die wasserstoffreiche Fraktion (25) wird als Heizmedium im Vorwärmer (1) verbrannt und die kohlenstoffreiche Fraktion (26) prozeßseitig dem Vorwärmer (1) zugeführt. Produkte des Verfahrens sind Methanol (28) und Kohlenmonoxid (17). Teile des Methanols (29) und des Kohlenmonoxids (17) können beispielsweise in einer nachfolgenden Essigsäure-Anlage (27) direkt weiterverarbeitet werden.
Essigsäure wird heutzutage üblicherweise durch katalytische Carbonylierung von Methanol hergestellt, wie z.B. in den Verfahren von BP Chemicals oder Celanese. Zusätzliche Anwendungsbereiche für Kohlenmonoxid sind denkbar.
Die einzige Emissionsquelle für Kohlendioxid ist das Rauchgas (31) des Vorwärmers (1) Nicht dargestellt ist die optionale Verwendung eines Vorreaktors, der zwischen den Stufen des Vorwärmers (1) eingesetzt wird.

### Beispiel:

Das folgende Beispiel, das teilweise berechnet ist, hat die Erzeugung von 2500 Tagestonnen Methanol und 586 Tagestonnen CO (98,8 %ig) zum Ziel. Die Erzeugung des Synthesegases erfolgt in einer katalytischen partiellen Oxidation. Dem Synthesegasreaktor (5) ist ein Vorreaktor vorgeschaltet, der optional ist und die höheren Kohlenwasserstoffe des Erdgases in Methan, Kohlendioxid und Wasserstoff umwandelt. Dieses vorreagierte Gas läßt sich ohne Crackreaktion beliebig hoch vorwärmen.

Die Zerlegung des kohlendioxid-freien Gasstromes (15) erfolgt in einer Tieftemperaturzerlegung (16). Das Purgegas (23) der Methanolsynthese (22) wird in einer Membrananlage (24) zerlegt. Die Kompressoren sowie alle wesentlichen Gebläse und Pumpen werden mittels Dampfturbinen angetrieben. Sowohl der Turbinenantriebsdampf als auch der Prozeßdampf werden aus Abwärme des Prozesses gewonnen. Der einzige Auslass für CO₂ ist das Rauchgas (31) des Vorwärmers (1).

Man arbeitet mit einer Anlage, die weitgehend der Zeichnung entspricht. Im Vorwärmer (1) werden 4444 kmol/h Erdgas und 6885 kmol/h Wasserdampf in einer ersten Stufe des Vorwärmers auf 500°C vorgewärmt. Nach Reaktion des vorgewärmten Einsatzgasstromes im Vorreaktor wird dem Austrittsgas Restgas aus der Leitung (26) und 528 kmol/h CO₂ aus Leitung (12) zugemischt und im Vorwärmer (1) auf 600°C vorgewärmt.

Das Erdgas enthält neben Methan 3,91 Mol % C₂H₆, 0,03 Mol % C₃H₈, 0,59 Mol % CO₂ und 0,08 Mol % N₂. Der katalytischen, partiellen Oxidation (5) werden außer dem Austrittsgas aus dem Vorreaktor 2556 kmol/h Sauerstoff (Reinheit 99,5 Mol %) zugeführt.

Weitere Einzelheiten ergeben sich aus der nachfolgenden Tabelle, wobei für verschiedene Leitungen die Komponenten des jeweiligen Gemisches in Mol % und kmol/h angegeben sind.

| **Leitung** | 9 | | 10 | | 19 | | 20 | | 21 | |
|---|---|---|---|---|---|---|---|---|---|---|
| | Mol % | kmol/h | Mol % | kmol/h | Mol % | kmol/h | Mol % | kmol/h | Mol % | kmol/h |
| CO₂ | 7,67 | 1662 | 10,87 | 528 | - | - | - | - | 8,87 | 1125 |
| CO | 16,90 | 3663 | 24,09 | 1170 | 2,91 | 26 | 2,92 | 68 | 20,18 | 2560 |
| H₂ | 43,94 | 9527 | 62,64 | 3042 | 94,30 | 843 | 94,30 | 2199 | 68,45 | 8683 |
| CH₄ | 1,25 | 272 | 1,79 | 87 | 2,68 | 24 | 2,70 | 63 | 1,95 | 248 |
| N₂ und Ar | 0,24 | 52 | 0,35 | 17 | 0,11 | 1 | 0,09 | 2 | 0,30 | 38 |
| CH₃OH | - | - | - | - | - | - | - | - | - | - |
| H₂O | 30,00 | 6504 | 0,25 | 12 | - | - | - | - | 0,25 | 32 |
| Gesamtmenge (kmol/h) | | 21680 | | 4856 | | 894 | | 2332 | | 12686 |
| Temperatur (°C) | 960 | | 40 | | 40 | | 40 | | 40 | |
| Druck (bar a) | 34 | | 31 | | 29 | | 29 | | 29 | |

Die Stöchiometriezahl S beträgt im rohen Synthesegas in der Leitung (9) S = 1,48 und im Gas der Leitung (21) S = 2,05 .

| **Leitung** | 23 | | 25 | | 26 | | 31 | |
|---|---|---|---|---|---|---|---|---|
| | Mol % | kmol/h | Mol % | kmol/h | Mol % | kmol/h | Mol % | kmol/h |
| CO₂ | 11,10 | 195 | 2,44 | 25 | 23,06 | 169 | 1,52 | 94 |
| CO | 3,64 | 64 | 0,20 | 2 | 8,59 | 63 | - | - |
| H₂ | 69,65 | 1223 | 95,50 | 978 | 33,42 | 245 | - | - |
| CH₄ | 12,98 | 228 | 1,56 | 16 | 28,92 | 212 | - | - |
| N₂ und Ar | 2,05 | 36 | 0,10 | 1 | 4,91 | 36 | 66,46 | 4118 |
| CH₃OH | 0,51 | 9 | 0,20 | 2 | 0,95 | 7 | - | - |
| H₂O | 0,06 | 1 | 0,01 | 0,1 | 0,14 | 1 | 30,42 | 1885 |
| O₂ | - | - | - | - | - | - | 1,60 | 99 |
| Gesamtmenge (kmol/h) | | 1756 | | 1024 | | 733 | | 6196 |
| Temperatur (°C) | 40 | | 40 | | 40 | | 190 | |
| Druck (bar a) | 78 | | 5 | | 77 | | 1 | |

## Patentansprüche

1. Verfahren zur Erzeugung von Methanol aus flüssigen oder gasförmigen Einsatzstoffen, wobei man aus diesen Einsatzstoffen unter Zugabe von Sauerstoff und Wasserdampf mittels katalytischer oder nichtkatalytischer partieller Oxidation ein Synthesegas (9) herstellt, **dadurch gekennzeichnet,**
**dass** ein Teilstrom (10) des Synthesegases abgezweigt und das darin enthaltene Kohlendioxid durch eine Kohlendioxid-Abtrennung (11) entfernt wird, dass dieses Kohlendioxid ganz oder teilweise in die Synthesegaserzeugung (5) zurückgeführt wird, dass das aus der Kohlendioxid-Abtrennung (11) abgezogene kohlendioxidfreie Gas einer Trennung (16) unterworfen wird, wobei Kohlenmonoxid produziert wird und der abgetrennte Wasserstoff dem Synthesegas ganz oder teilweise wieder zugeführt wird und dass das mit Wasserstoff angereicherte Synthesegas in der Methanolsynthese (22) zu Methanol und Purgegas umgesetzt wird.

2. Verfahren nach Anspruch 1, **dadurch gekennzeichnet, dass** das aus der Kohlendioxid-Abtrennung (11) abgezogene kohlendioxidfreie Gas mittels kryogener Trennung in Kohlenmonoxid und Wasserstoff getrennt wird.

3. Verfahren nach Anspruch 1, **dadurch gekennzeichnet, dass** das aus der Kohlendioxid-Wäsche (11) abgezogene kohlendioxidfreie Gas mittels Membranen in Kohlenmonoxid und Wasserstoff getrennt wird.

4. Verfahren nach Anspruch 1, **dadurch gekennzeichnet, dass** ein Teil des Kohlenmonoxids aus der Gastrennung (16) mit einem Teil des Methanols in einer Anlage (27) zu einem anderen Produkt, z.B. Essigsäure, weiterverarbeitet wird.

5. Verfahren nach Anspruch 1, **dadurch gekennzeichnet, dass** ein Teil des aus der Gastrennung (16) entstehenden Wasserstoffs zur Unterfeuerung des Vorwärmers (1) verwendet wird.

6. Verfahren nach Anspruch 1, **dadurch gekennzeichnet, dass** das Purgegas (23) der Methanolsynthese (22) mittels einer Gastrennung (24), in eine wasserstoffreiche (25) und eine kohlenstoffreiche (26) Fraktion aufgetrennt wird und die wasserstoffreiche Fraktion (25) zur Unterfeuerung des Vorwärmers (1) verwendet wird und die kohlenstoffreiche Fraktion (26) zur partiellen Oxidation zurückgeführt wird.

7. Verfahren nach Anspruch 1, **dadurch gekennzeichnet, dass** der partiellen, katalytischen Oxidation ein mit hochaktivem Nickelkatalysator gefüllter Vorreaktor vorgeschaltet ist.

8. Verfahren nach Anspruch 1, **dadurch gekennzeichnet, dass** Kohlendioxid aus externen Quellen über Leitung (14) dem Reaktor (5) zugeführt wird.

## Claims

1. Process for the production of methanol from liquid or gaseous starting materials in which a synthesis gas (9) is produced from these starting materials by catalytic or non-catalytic partial oxidation with addition of oxygen and steam, **characterized in that** a sub-stream (10) of the synthesis gas is branched off, and the carbon dioxide present therein is removed by carbon dioxide separation (11), **in that** all or some of this carbon dioxide is fed back into the synthesis gas production (5), **in that** the carbon dioxide-free gas removed from the carbon dioxide separation (11) is subjected to separation (16), producing carbon monoxide, and all or some of the hydrogen separated off is fed back to the synthesis gas, and **in that** the hydrogen-enriched synthesis gas is converted into methanol and purge gas in the synthesis (22) of methanol.

2. Process according to Claim 1, **characterized in that** the carbon dioxide-free gas removed from the carbon dioxide separation (11) is separated into carbon monoxide and hydrogen by means of cryogenic separation.

3. Process according to Claim 1, **characterized in that** the carbon dioxide-free gas removed from the carbon dioxide wash (11) is separated into carbon monoxide and hydrogen by means of membranes.

4. Process according to Claim 1, **characterized in that** some of the carbon monoxide from the gas separation (16) is processed further with some of the methanol in a plant (27) to give another product, for example acetic acid.

5. Process according to Claim 1, **characterized in that** some of the hydrogen formed from the gas separation (16) is used for heating the preheater (1) from below.

6. Process according to Claim 1, **characterized in that** the purge gas (23) in the methanol synthesis (22) is separated into a hydrogen-rich fraction (25) and a carbon-rich fraction (26) by means of a gas separation (24), and the hydrogen-rich fraction (25) is used for heating the preheater (1) from below and the carbon-rich fraction (26) is fed back for partial oxidation.

7. Process according to Claim 1, **characterized in that** a pre-reactor filled with highly active nickel catalyst is installed upstream of the partial catalytic oxidation.

8. Process according to Claim 1, **characterized in that** carbon dioxide from external sources is fed to the reactor (5) via line (14).

## Revendications

1. Procédé pour la production de méthanol à partir de substances de départ liquides ou gazeuses, dans lequel, à partir de ces substances de départ, par addition d'oxygène et de vapeur d'eau, au moyen d'une oxydation partielle catalytique ou non catalytique, on prépare un gaz de synthèse (9), **caractérisé :**
**en ce qu'**on dérive un courant partiel (10) du gaz de synthèse et on élimine le dioxyde de carbone qui y est contenu via une séparation du dioxyde de carbone (11) ; en ce qu'on renvoie ce dioxyde de carbone, en tout ou en partie, dans la production du gaz de synthèse (5) ; en ce qu'on soumet à une séparation (16) le gaz exempt de dioxyde de carbone issu de la séparation du dioxyde de carbone (11), si bien que l'on obtient du monoxyde de carbone, l'hydrogène séparé étant renvoyé en tout ou en partie au gaz de synthèse ; et en ce qu'on transforme le gaz de synthèse enrichi en hydrogène, dans la synthèse du méthanol (22), en méthanol et en gaz de purge.

2. Procédé selon la revendication 1, **caractérisé en ce que** le gaz exempt de dioxyde de carbone issu de la séparation du dioxyde de carbone (11) est séparé en monoxyde de carbone et en hydrogène au moyen d'une séparation cryogénique.

3. Procédé selon la revendication 1, **caractérisé en ce que** le gaz exempt de dioxyde de carbone issu du lavage de dioxyde de carbone (11) est séparé en monoxyde de carbone et en hydrogène au moyen de membranes.

4. Procédé selon la revendication 1, **caractérisé en ce qu'**une partie du monoxyde de carbone provenant de la séparation du gaz (16) est soumis à un traitement ultérieur avec une partie du méthanol dans une installation (27) pour obtenir un autre produit, par exemple de l'acide acétique.

5. Procédé selon la revendication 1, **caractérisé en ce qu'**on utilise une partie de l'hydrogène que l'on obtient à partir de la séparation du gaz (16) pour le foyer inférieur du préchauffeur (1).

6. Procédé selon la revendication 1, **caractérisé en ce que** le gaz de purge (23) de la synthèse du méthanol (22) est séparé, au moyen d'une séparation de gaz (24), en une fraction riche en hydrogène (25) et en une fraction riche en carbone (26), la fraction riche en hydrogène (25) étant utilisée pour le foyer inférieur du préchauffeur (1) et la fraction riche en carbone (26) étant recyclée à des fins d'oxydation partielle.

7. Procédé selon la revendication 1, **caractérisé en ce qu'**un préréacteur rempli d'un catalyseur très actif à base de nickel est monté en amont de l'oxydation catalytique partielle.

8. Procédé selon la revendication 1, **caractérisé en ce que** le dioxyde de carbone est acheminé au réacteur (5) à partir de sources externes, via un conduit (14).
